# EUROPEAN PATENT SPECIFICATION

(11) **EP 2 978 493 B1**
(45) Date of publication and mention of the grant of the patent: **10.10.2018**
(21) Application number: 14714208.7
(22) Date of filing: 21.03.2014
(51) Int. Cl.: A61N 5/06, D03D 1/00, D03D 15/00, F21V 8/00, G02B 6/36

(54) **MEDICAL DEVICE AND ITS PREPARATION METHOD**
MEDIZINISCHE VORRICHTUNG UND DEREN HERSTELLUNGSVERFAHREN
DISPOSITIF MÉDICAL ET SON PROCÉDÉ DE PRÉPARATION

(30) Priority: 26.03.2013 EP 13305373
(43) Date of publication of application: 03.02.2016
(73) Proprietor: Institut National de la Santé et de la Recherche Médicale (INSERM), 75013 Paris (FR); Université de Lille 2 Droit et Santé, 59800 Lille (FR); Centre Hospitalier Régional et Universitaire de Lille, 59000 Lille (FR)
(72) Inventor: MORDON, Serge, 59037 Lille Cedex (FR)
(74) Representative: Cabinet Plasseraud
(86) International application number: PCT/EP2014/055770
(87) International publication number: WO 2014/154595

(56) References cited:
- US-A- 5 339 223
- US-A1- 2006 144 460
- US-A1- 2007 281 155

## Description

### FIELD OF THE INVENTION

The present invention relates to a medical device and its its preparation method.

### BACKGROUND OF THE INVENTION

Photodynamic therapy (PDT) is a non-thermal technique which can be used to produce localised tissue necrosis. This requires activating a pre-administered photosensitizer with light of a specific wavelength to form a cytotoxic species from molecular oxygen (mostly singlet oxygen). For a photodynamic reaction to occur, the photosensitising agent, activating light and oxygen must be present in sufficient amounts.

The therapeutic effect of photodynamic therapy depends on a combination of parameters that include drug dose, drug-light interval, oxygen and light fluence rate. It also varies according to the wavelength distribution of the light source. Finally, a homogeneous and reproducible fluence rate delivery during clinical PDT is determinant in preventing under- or overtreatment. In Dermatology, topical PDT has been carried out with a wide variety of light sources delivering a broad range of light doses. Irradiance is usually limited to less than 100 mW.cm⁻².

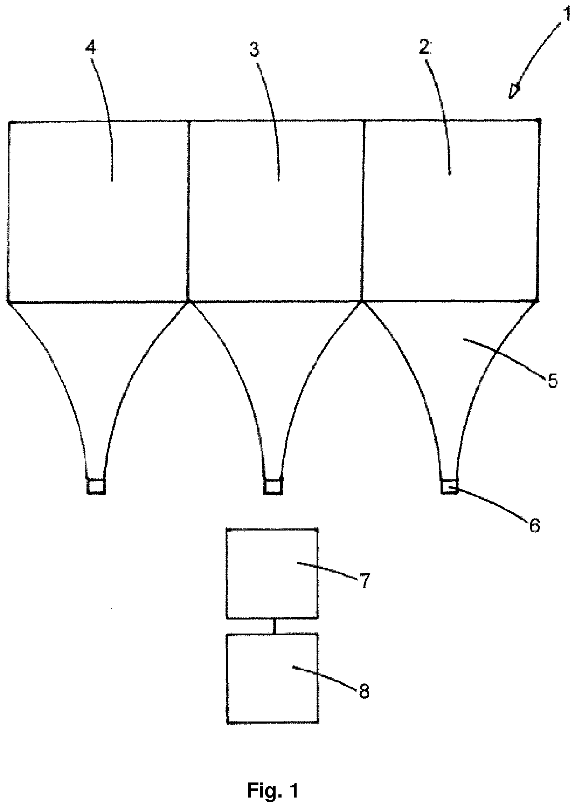

Light-emitting diodes (LEDs) are now considered as an appropriate light source for PDT. Indeed, LEDs have a relatively narrow bandwidth (usually 20 to 30 nm) and are available in a wide range of wavelengths. LED systems for Methyl aminolevulinate PDT (MAL-PDT) such as Aktilite® CL 16 and Aktilite® CL 128 (Metvix, Galderma) are now mainly used. The Aktilite® CL 16 treats areas of skin measuring 40 x 50 mm whereas the Aktilite® CL 128 treats larger areas (80 x 180 mm) (Figure 1). They provide light doses of 37 J.cm⁻² required for the optimal activation of Metvix. Fluence rate varies between 70 and 100 mW.cm⁻², for an irradiation time varying between 6 and 10 minutes. However even commercial systems, such as Aktilite® CL 16, do not deliver a uniform light distribution (Moseley, 2005). In the case of the CL 16, the irradiance may be as low as 38 % of the central area at a distance of only 2 cm. These measurements were made on a flat surface. The heterogeneity is even greater during illumination of curved surfaces (face or scalp).

Also a homogeneous and reproducible fluence delivery rate during clinical photodynamic therapy plays a determinant role in preventing under- or over-treatment. Photodynamic therapy applied in Dermatology has been carried out with a wide variety of light sources delivering a broad range of more or less adapted light doses. Due to the complexity of human anatomy, such as the human face and also vulval, and perianal areas, these light sources do not in fact deliver a uniform light distribution to the skin. Therefore, the development of flexible light sources considerably improves the homogeneity of light delivery. The integration of plastic optical fibres into textile structures offers an interesting alternative to rigid light emitters.

In dermatology, the clinical use of 5-aminolaevulinic acid (ALA) induced protoporphyrin IX (PPIX) for photodynamic therapy is proposed for non-melanoma skin cancer treatment. However, this treatment is painful limiting the suitability of photodynamic therapy as a treatment of first choice. Patients report a burning or tingling sensation that sometimes leads to need for local anesthesia or termination of therapy. Especially treating extensive field cancerization with actinic keratosis in the face and scalp region is painful for the patient.

One way to reduce the pain consists in light dose fractionation. Irradiation is interrupted at a particular point for a period of time. There is therefore a succession of illumination periods and of rest periods. Besides, light fractionation to increases the efficacy: light fractionation produces more necrosis than with the same light dose delivered without rest periods.

Conventional light sources necessary for photodynamic therapy are expensive. Therefore an inactive or rest period is a waste of medical means.

Consequently the use of PDT has largely been limited to hospital outpatient services where costs can be high and the service inconvenient for the patient.

New concepts in illumination, such as ambulatory PDT or daylight illumination might contribute to the further acceptance of this method.

Additionally, actinic keratosis (AK) are scaly or crusty growths (lesions) caused by damage from the sun's ultraviolet rays (UVR). Actinic Keratosis is also known as solar keratosis. Untreated actinic keratosis can advance to squamous cell carcinoma (SCC), the second most common form of skin cancer. Treatment options include ablative (destructive) therapies such as cryosurgery, curettage with electrosurgery, and photodynamic therapy. Topical photodynamic therapy for actinic keratosis is now a well established treatment modality, with two drugs registered for this indication. In the last years, new formulations have been developed, which promise a further improvement of actinic keratosis treatment. Photodynamic therapy is well tolerated, has excellent cosmetic results, and has reported cure rates between 69 and 93 %, with fewer side effects compared to the other treatment options. Presently, a flat LED panel is used as light source.

A conventional protocol using Metvix® (methyl aminolevulinate) consists in having Metvix® specifically absorbed into the altered skin cells of these lesions. Metvix® causes compounds called porphyrins to accumulate and be absorbed selectively by the actinic keratosis. Metvix® is applied to the lesions to be treated. The lesion is covered with a dressing. There is a 3 hour waiting period for the Metvix® cream to be absorbed. After 3 hours, Metvix® is washed off and immediately illuminated with a red light, the intensity and time exposed to red light depends on the type of lesions that are treated. Light exposure can last between 8-20 minutes. The illumination is not homogeneous since a LED panel is used.

The size and the design of the led panel are not appropriate for bald scalps. Since the treatment is performed in a short period of time, the treatment is usually very painful.

US 2006/0257095 discloses a light diffuser for photodynamic therapy comprising a flat flexible support and a plurality of light-emitting elements affixed thereto. Each light-emitting element of the light diffuser further comprises a light-supplying optical fiber, said optical fiber not being a woven or integral part of said flexible support. To each optical fiber there is associated exactly one light-emitting element that comprises at least one output zone formed by a local curvature of the optical fiber by either a fixation stitch or at least one loop. The light diffusers are flexible, and may be used on complex body surfaces.

However, in a publication in the Journal of Biomedical Optics 12 3, 034024 May/June 2007, the inventors stated that a power setting of 100 mW increases the temperature of the textile diffuser surface of up to 27°C, and a power of 1 W heated the textile diffuser from 22°C to more than 30°C in less than 1 min, and induced a mean temperature on the surface of 36°C and maximum values above 40°C after 5 min. Furthermore, illumination is not homogeneous as shown on a figure representing a 3-D image and a brightness distribution histogram of the calculated luminous textile surface. The emission of each point can easily be recognized on a figure hereafter showing the light emitted by the above light diffuser. The textile diffuser achieved an irradiance of 3.6 mW/cm2. Development of this light diffuser was therefore stopped.

Textile light diffusers for light therapy are also envisaged in the following documents: US 5 339 223 dealing with phototherapy of neonatal jaundice; US 2007 288 071 discussing PDT treatment of skin problems; and WO 2012 098 488 referring to dermatological phototherapy.

One challenge in order to ensure the development of such treatment is to guarantee a uniform light illumination of the skin due to the complexity of the human anatomy and an important irradiance while avoiding excessive increase of temperature.

Now the present inventors have developed a new medical device which allows a continuous use of light sources and allows delivering a uniform light distribution to complex body shapes and high fluence rate of illumination.

Additionally, since the efficacy of photodynamic therapy (PDT) depends on the amount of singlet oxygen which itself depends on the amount of pre-administered photosensitizer because for a photodynamic reaction to occur, the photosensitising agent, activating light and oxygen must be present in sufficient amounts, it is important to determine the amount of active photosensitising agent remaining at the site of action. In fact degradation of the photosensitiser occurs during treatment and this effect is named photobleaching.

The measurement of photobleaching allows knowing the efficacy of photodynamic therapy and adjusting the light treatment. The principle of measurement is to estimate the amount of singlet oxygen produced by photodynamic therapy by measuring the rate of photobleaching of the photosensitizer by measuring the fluorescence. For example for protoporphyrin IX, an excitation light at 405 nm and collecting the fluorescence at 630 nm can be used.

The invention is defined in the claims, other methods, embodiments and examples being mentioned for illustrative purposes only.

### SUMMARY OF THE INVENTION

A subject of the present application is therefore a medical device comprising a flexible light source wherein said flexible light source comprises three individually manageable areas of light emission and wherein each area comprises a light diffuser textile comprising optical fibres providing side diffusion of a light.

Whereas usually in optical fibres light is guided down the core of the fibre by a coating named optical cladding having a refractive index lower than the refractive index of the core, that traps light in the core through total internal reflection, in the present invention the optical fibres are bent such that a critical angle is exceeded whereby light escapes from the core of the fibres providing side diffusion of the light.

### DESCRIPTION OF THE PREFERRED EMBODIMENTS

More particularly, integration of flexible optical fibres into textile tissue structures provides a light diffuser textile used in the invention.

In one embodiment of light diffuser textile, a plain weave of optical fibres and other possibly coated textile fibres is used. The light travelling through the optical fibres is emitted through scratches, pierced by mechanical indentation (toothed roll). Scratches may also be obtained by projecting particles at the cladding or by chemical treatment (solvent action to locally dissolve the cladding). These kinds of indentations are possible with the polymer optical fibres such as polymethyl methacrylate optical fibres.

In another embodiment of light diffuser textile, the polymer optical fibres emit light without indentation of the surface fibre or cladding. For instance, a light emitting panel made from one or more layers of polymer optical fibres woven into a sheet (plain weave) and coated with diffusive material in order to better diffuse laterally emitted light may be used. The plain weave structure of the fabric, in this case, enables the bends of the woven optical fibres allowing light emission laterally. The diffusive layer improves the homogeneity of light distribution. Such an illuminating panel is for example available from Lumitex Company.

In another embodiment of light diffuser textile, an embroidery-based light diffuser textile consists of a dense woven substrate in which polymer optical fibres are fixed using conventional yarn. The polymer optical fibres forms random bends and loops leading to macrobending of the fibre from which light can escape. Such a textile construction maybe composed of 178 polymer optical fibres (polymethyl methacrylate, diameter of 175 µm) providing a flexible diffuser about 2 mm in height and delivering a round luminous area of about 11 cm².

The light diffuser textile of the invention is obtained by weaving. The warp provides the skeleton of the textile whereas the weft comprises the optical fibres. The warp preferably comprises polyester yarns whereas the weft preferably comprises polymethyl methacrylate optical fibres.

Examples of suitable polyester yarns are copolyester yarns are commercialised by Sinterama, for example, commercial reference LAST®.

The linear mass density of polyester yarns may be in a range of 50 to 500 dTex, preferably of 100 to 400 dTex, particularly of 300 to 370 dTex, and more particularly of approximately 330 dTex.

When polyester yarns are made of continuous microfibres, light delivering is increased.

Polyester yarns must be strong enough for allowing sufficient bending of the optical fibres to exceed a critical angle whereby light escapes from the core of the fibres providing side diffusion of the light.

Examples of suitable polymethyl methacrylate optical fibres are commercialised by Toray (Japan), for example with commercial references RAYTELA®, PG series. The polymethyl methacrylate core thereof is surrounded by a fluorinated polymer cladding.

The diameter (including the cladding) of optical fibres, preferably polymethyl methacrylate optical fibres, may be in a range of 750 to 100 µM, preferably of 500 to 200 µM, particularly of 300 to 200 µM, more particularly of 270 to 230 µM, and very particularly of approximately 250 µM. The smaller the diameter of the optical fibres, the more a light diffuser textile thus manufactured is flexible.

The smaller the thickness of the cladding, the more a light diffuser textile thus manufactured is efficient. An optical fibre with a very thin cladding, for example about 1/50 of the overall diameter, is preferred for improving the efficiency of the light collection.

Warp yarns of a preferred light diffuser textile of the invention have a density of about 20 cm⁻¹. Said density may be determined for example by optical count.

Weft density varies according to weave and may be determined for example by optical count.

Dimensions of a light diffuser textile of the invention, i.e. dimensions of the woven area, may vary in a broad range, only limited by the weaving material used. Typical dimensions of a light diffuser textile of the invention, are 21.5 cm (weft, named width, W) x 5 cm (warp, named length, L). Other preferred dimensions are for example 25 cm x 15 cm, 30 cm x 20 cm or 15 cm x 10 cm. Since a medical device of the invention comprises two or more light diffuser textiles, the surface of the medical device will be for example twice or three times the surface of an individual light diffuser textile.

In order to connect the fabric to the light source, the total length of optical fibres is longer than the length actually woven in the woven area. For example 15 cm to 1 m is free (non-woven) on one side or both sides of the woven area, preferably of sites.

Particularly preferred textiles of the invention are obtained by mechanized weaving and are advantageously plain weave or satin weave. Examples of satin weave are satin weave 4 (SW4), satin weave 6 (SW6) and satin weave 8 (SW8). Combinations of patterns using different weaves are preferred because they allow providing a flexible fabric having a very uniform distribution of light.

A flexible light source of a medical device of the invention comprises three individually manageable areas of light emission, each area comprising a light diffuser textile. Therefore each individually manageable area may be used for light emission independently of each other area.

Preferably, the different areas of a given medical device have an identical or similar surface. For example in a case of a flexible light source having three individually manageable areas, each area of light emission represents about one third of the entire surface of the flexible light source.

Under preferred conditions for implementing the invention, a single light-emitting device, preferably comprising a laser, particularly a diode laser, may be connected to each and every area of light emission. Therefore, since the different areas of light emission are individually manageable, one area is active whereas the other areas are at rest. Furthermore the same light-emitting device may later be used for illuminating another area. Accordingly although the light emitting device may be continuously working, only a part of the medical fabric of the invention is active whereas another or other parts are at rest (inactive).

In view of easily connecting a light-emitting device, the optical fibres constituting the weft of each light diffuser textile are preferably collected as bundles and said bundles are further provided with a port of entry (input) of light such as optical fibre connectors.

A subject of the present invention is also a medical apparatus for photodynamic therapy comprising an above medical fabric and a light-emitting device suitable for photodynamic therapy, preferably comprising diodes, whereby the flexible light source of the invention provides side diffusion of light.

A subject of the present invention is also a process for the manufacture of an above medical device of the invention comprising the steps consisting in manufacturing three light diffuser textiles obtained by
- providing a weft comprising optical fibres,
- interlacing at right angles a warp preferably comprising polyester yarns and said interlacing providing the bending of said weft such that a critical angle allowing side emission of light is exceeded, and
- preparing a flexible light source comprising said three light diffuser textiles.

For example a medical device comprising three light diffuser textiles may be prepared by bundling the optical fibres into three groups of adjacent fibres.

Three independent light diffuser textiles may also be manufactured and assembled as a single flexible light source for a medical device.

In preferential conditions of implementation of the invention, optical fibres constituting the weft of each light diffuser textile are further provided with one or several ports of entry of light for easy connection to a light emitting device.

A subject of the present invention is also a bifunctional medical device comprising a flexible light source wherein said flexible light source comprises
- three individually manageable areas of light emission and wherein each area comprises a light diffuser textile comprising optical fibres providing side diffusion of a light and
- said three individually manageable areas of light emission comprising optical fibres providing side absorption of a light.

An emission of light is for example produced by a photosensitiser substance that has absorbed light or other electromagnetic radiation. This emission of light may be absorbed by the optical fibres providing side absorption of a light of the invention. For example protoporphyrin IX may receive an excitation light at 405 nm and in turn provide emission of fluorescence at 630 nm which may be absorbed by the optical fibres.

The general structure of the above bifunctional medical device is similar to the structure of the above medical device comprising a flexible light source wherein said flexible light source comprises three individually manageable areas of light emission and wherein each area comprises a light diffuser textile comprising optical fibres providing side diffusion of a light, with a few particularities.

A given area may be provided with a single bundle having a connector. The connector may be alternatively connected either to a light-emitting device suitable for photodynamic therapy or to a photodetector. A given area may also be provided with a pair of bundles or more, each bundle corresponding to different optical fibres being specialised either for therapy or for detection of bleaching. Additionally a third bundle may be used for providing a light with a wavelength different of the therapeutic wavelength for a better detection of bleaching.

In an individually manageable area of light emission, the ratio fibres used for detection / fibres used for therapy, may be in the range 1/1 to 1/20, preferably in the range 1/2 to 1/15, more preferably in the range 1/3 to 1/15. In such a case, fibres used for detection are preferably well spread over the entire surface of the area, more preferably distributed in a balanced way, for example one fibre for detection every five fibres used for therapy.

Suitable photodetectors are for example commercialised by Siemens.

Preferred conditions for implementing the medical devices without optical fibres dedicated to the detection described above also apply to the above medical devices comprising such fibres.

The medical devices according to the invention have advantageous properties. They constitute flexible and homogenous light diffusers providing side-emission of light. Because of their flexibility, they are easily adapted to human curves. They offer a cheap alternative as light source for photodynamic therapy. In addition to a uniform light illumination of the skin, they provide strong and homogenous irradiance, around 20 mW/cm² while avoiding excessive increase of temperature.

The achieved homogeneity is much better than results obtained with commercially available LED panels. With a flat panel of LEDs such as described in US 4,907,132, some surfaces of skin are close to the light emitters whereas other are remote. Unlike LED panels, a light diffuser of the invention is flexible and can closely follow the surface to be treated. In contrast with US 2006/0257095, a light diffuser of the invention provides a much more important irradiance while avoiding excessive increase of temperature.

Another advantage is that light diffusers of the invention act as a waveguide, and thus the light source is separated from the light emitting device. Hence, with a same light emitting device it is possible to diffuse light of different wavelengths by simply changing the source.

The medical devices according to the invention further provided with one or more bundles specialised for photo detection are bifunctional since they allow treatment in combination with a photosensitizer and additionally they allow assessing the level of bleaching of the photosensitizer.

Because of the above properties the medical devices comprising a flexible light source according to the invention are used as light diffusers for photodynamic therapy.

Some embodiments also may be used as light diffusers for photodynamic therapy and detectors of bleaching of a photosensitiser.

Therefore the subject of the invention is a medical apparatus for photodynamic therapy comprising a medical device as disclosed above and a light-emitting device suitable for photodynamic therapy such as those previously mentioned. A further subject of the invention is such a medical apparatus for photodynamic therapy further comprising a photodetector.

Light-emitting devices suitable for photodynamic therapy are for example lasers and preferably diode lasers providing light having a wavelength of 400 to 800 nanometres for example depending on the active ingredient used. Such light emitting devices are for example commercialised by OMNILUX (http://www.omnilux.co.uk/).

Suitable photosensitizers for photodynamic therapy are for example hematoporphyrine (630 nm), meta-tetra hydroxyphenyl chlorine, (652 nm), benzoporphyrine (690 nm), bacteriopheophorbides (753 nm), methyl aminolevulinate (405 to 670 nm). Excitation wavelengths are given between brackets.

The subject of the invention is a medical apparatus as defined above further comprising means for managing light emission by a medical device of the invention, independently for each of the three areas. Means for managing light emission comprise a computer. Data specific for a treatment of a patient may therefore be provided to a computer which controls intensity, duration and frequency of irradiation. An example of treatment of actinic keratosis using a medical device comprising a flexible light source having 3 individually manageable areas in association with a photosensitizer is 30 cycles each cycle being a sequence consisting of 1 min illumination of area 1, then 1 min illumination of area 2, then 1 min illumination of area 3, then again 1 min illumination of area 1 etc. with an intensity of light (irradiance) of 10 mW/cm².

The invention will now be described by means of the following Examples

### DESCRIPTION OF THE DRAWINGS

Figure 1 schematizes an embodiment of a medical device of the invention comprising a flexible light source comprising three individually manageable light diffuser textiles.
Figure 2 schematizes a variant of Figure 1.
Figure 3 schematizes an embodiment of a medical device of the invention comprising a flexible light source comprising one bundle of optical fibres for therapy and one bundle of optical fibres for detection of bleaching.
Figure 4 shows structures of satin weave 4, 6 and 8 textiles.
Figure 5 illustrates an optical fibre bent such that a critical angle is exceeded whereby light escapes from the core of the fibre.
Figure 6 shows the structure of the specific light diffuser textile of example 1.

Figure 1 schematizes a medical device 1 of the invention comprising a flexible light source. The flexible light source 1 comprises three individual rectangular light diffuser textiles 2, 3, 4. Textiles 2, 3, 4 are the woven area of the medical device.

In order to connect each light diffuser textile 2, 3, 4 to a light source, a part of the length of the optical fibres is free. The free sections 5 of the optical fibres are provided on the same side of the flexible light source .The free sections 5 of the optical fibres are bundled and inserted into a brass ferrule 6 allowing optical connection to a light source 7. After gluing and cutting, the end of the optical fibres is highly polished such that an excellent surface is obtained. The three brass boxes 6 are used as ports of entry of light for optical fibre connectors provided on the light source 7. The light source 7 is managed by a computer 8 and provides light sequentially to each light diffuser textile 2, 3, 4 according to a sequence 2-3-4-2-3-4-2-3-4-2 etc. On figure 1, the ports are not connected to the optical fibre connectors of light source 7.

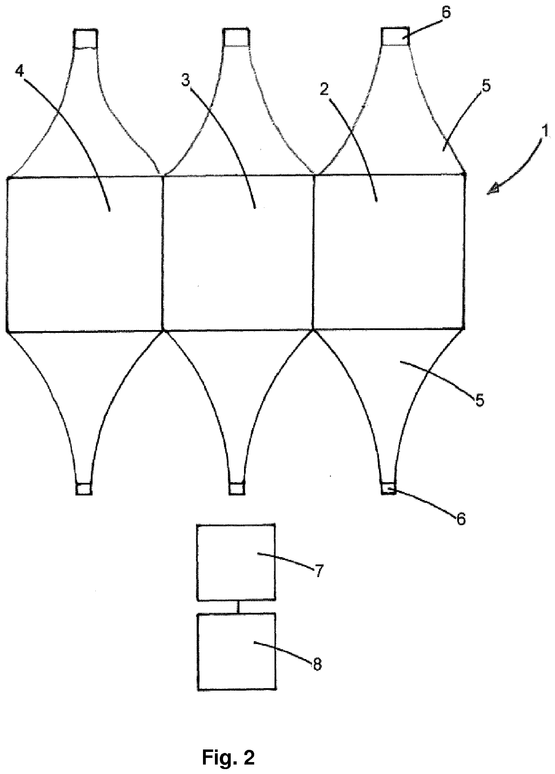

The medical device of figure 1 can be provided with light only from one side. Additionally, free sections 5 of the optical fibres may be shorter than those of the embodiment of figure 2.

Free sections 5 of the optical fibres of the embodiment of Figure 2 are provided on opposite sides of the flexible light source. Each flexible light source is provided with two bundles of free (non woven) optical fibres.

Accordingly, light may be provided at both ends of optical fibres. Long free sections 5 allow using a single light source 7. Schematic figure 2 does not respect the real sizes since free sections 5 should be longer for allowing connection to a single light source.

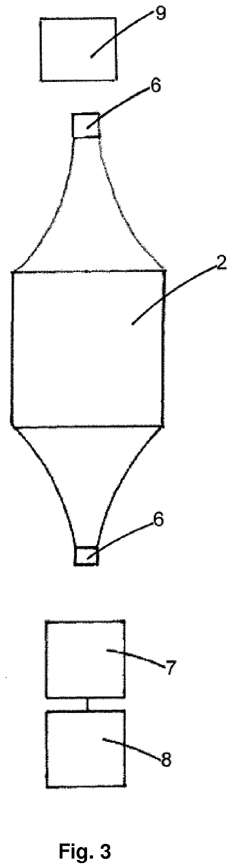

Figure 3 schematizes an embodiment of a medical device of the invention comprising a flexible light source comprising one bundle of optical fibres for therapy and one bundle of optical fibres for detection of bleaching. The bottom bundle collects optical fibres specifically used for therapy and the upper bundle collects optical fibres specifically used for detection of bleaching. The latter fibres are bundled into a highly polished brass ferrule 6 allowing optical connection to a photodetector 9. Light source 7 may provide two different wavelengths, one for therapy and one for greater accuracy with the detector after emission of light produced by a photosensitiser substance. In this schematic embodiment, the two bundles are provided on opposite sides of the textile. However both bundles may of course be provided on a same side.

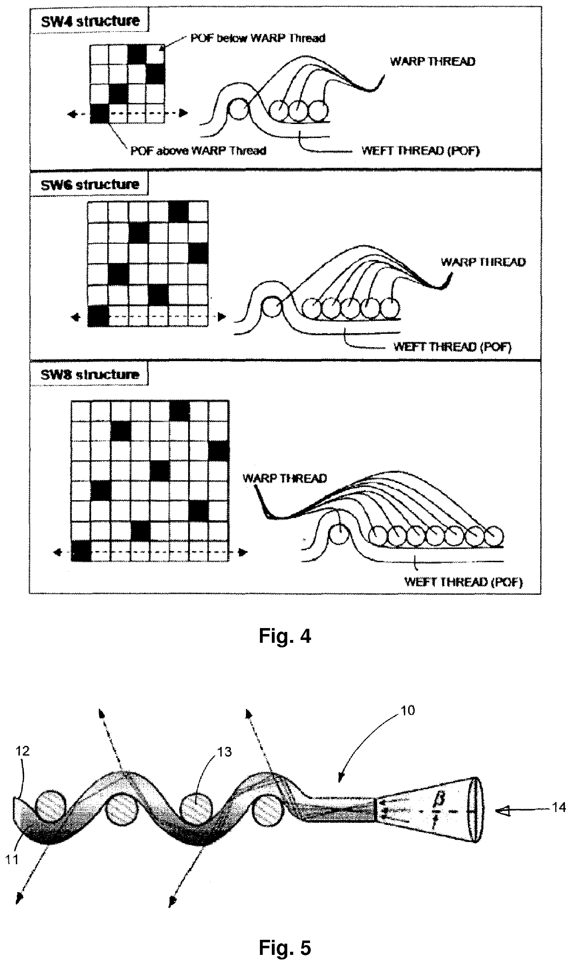

Figure 4 shows structures of satin weave 4, 6 and 8 textiles. POF means optical fibres.

Figure 5 shows one optical fibre 10 having a core 11 and a thin cladding 12 bent around four polyester fibres 13 such that a critical angle is exceeded, whereby light escapes according to the arrows from the core 11 of the fibre 10, providing side diffusion of light 14 coming from the right.

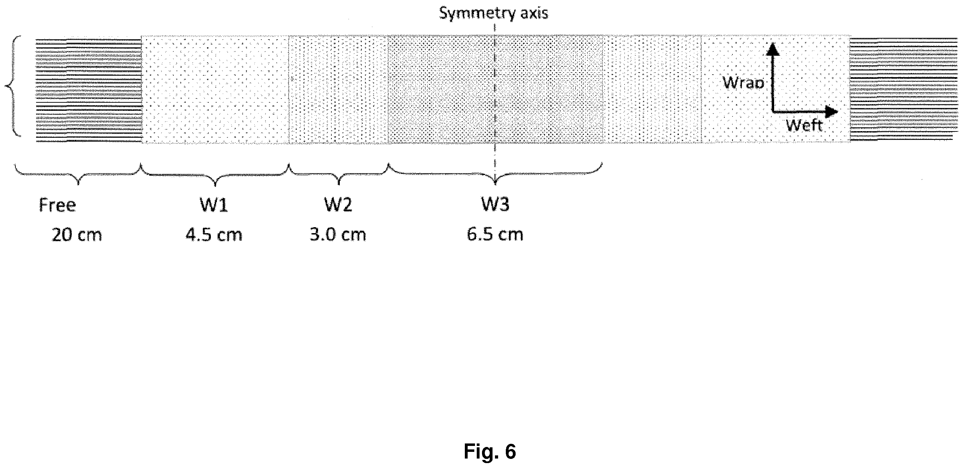

Figure 6 shows the structure of the specific light diffuser textile of example 1.

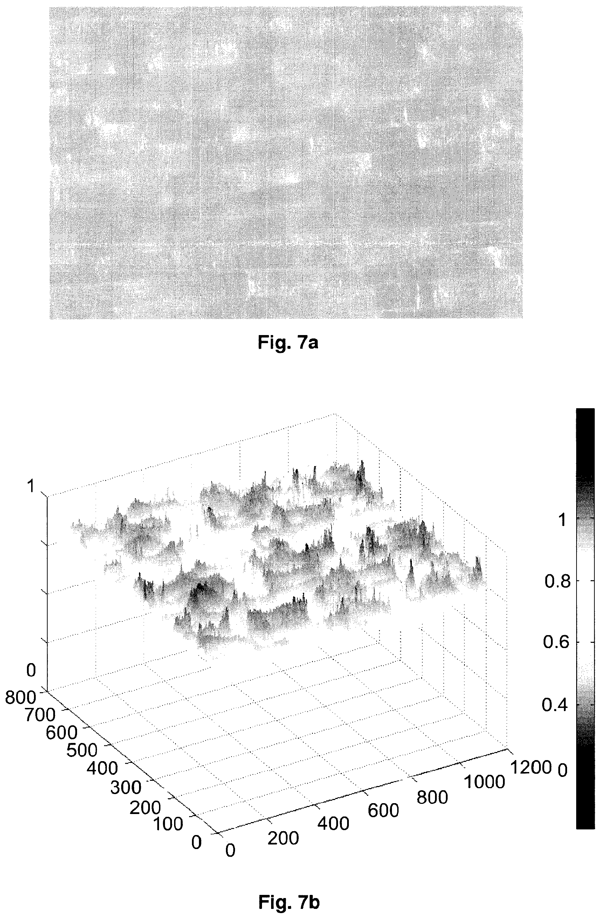

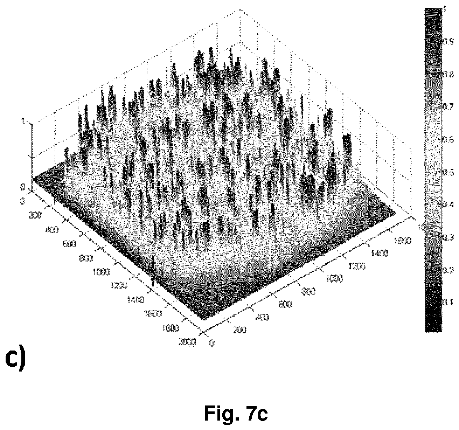

Figures 7 a and b illustrate the homogeneity of light emission with a flexible light source of the invention while Figure 7c illustrates the absence of homogeneity of light emission of the light diffuser of US 2006/0257095 with very sharp peaks. Figure 7a is a macroscopic photograph of the illuminated LEF : size : 1 cm x 1.5 cm). Figures 7b and 7c display the light distribution (intensity: arbitrary unit) measured on the surface.

From the left of the drawing, about 20 cm long free non-woven optical fibres are found, followed by a 21.5 cm long woven area comprising W1 weave (4.5 cm), W2 weave (3.0 cm), W3 weave (6.5 cm), W2 weave (3.0 cm), and W1 weave (4.5 cm). Wi patterns are explained hereafter. On the opposite side, the free other ends of the optical fibres are found. Their length is also about 20 cm. the woven area is 5 cm wide and comprises 187 weft optical fibres (density 37 fibres per cm).

The free optical fibres are to be bundled and inserted into a connector for connection to a light source.

### EXAMPLES

### Example 1: Manufacture of flexible light diffuser textiles

All flexible light diffuser textiles were woven using the hand weaving loom ARM B60 from Biglen (Switzerland).

The warp yarns are composed of 330 dTex polyester from Sinterama with a density of 20 cm⁻¹.

Toray Raytela® PG series Polymethyl methacrylate optical fibres with fluorinated polymer cladding (refractive index 1.41) are introduced as weft using a modified shuttle. The cladding diameter thereof is 250 µm.

Weft density varies according to weave and is determined by optical count. The dimension of the flexible light diffuser textile manufactured is 21.5 cm (weft, named width, W) x 15 cm (warp, named length, L). In order to connect the fabric to a light source, the total length of polymethyl methacrylate optical fibres is about 60 cm: 21.5 cm are weave and approximately 20 cm + 20 cm on each side of the woven area are free. The density of the optical fibres is 37 per cm.

Five samples have been woven: four samples made from basic weave and one sample of textile light diffuser with a weave pattern specifically developed for photodynamic therapy application.

The four samples are plain weave (PW), satin weave 4 (SW4), satin weave 6 (SW6) and satin weave 8 (SW8). The structure of the specific light diffuser textile is the following: The width of the light diffuser textile is composed of the following 5 areas with appropriate dimensions and structures of weaving: free optical fibres/4.5 cm W1/3.0 cm W2/6.5 cm W3/3.0 cm W2/4.5 cm W1/ free optical fibres. The five areas compensate attenuation of side emitted light and allow obtaining homogeneous light diffusers.

The weft density is 37 fibres /cm and the warp density 20 yarns / cm. The fabric comprises
- A first strip W1 comprising, in the longitudinal direction, three A patterns each consisting of one Satin 8 weave and three C patterns each consisting of four Satin weaves 4 arranged in a square, the patterns A and C being alternated;
- A second strip W2 comprising eight B patterns each consisting of one Satin weave 6 in the longitudinal direction;
- A third strip W3 comprising, in the longitudinal direction, three C patterns each consisting of four Satin weaves 4 arranged in a square and three A patterns each consisting of a Satin weave 8, patterns A and C being alternated. In addition, when the first strip has a pattern A, the third strip has a transverse pattern C. Patterns A and C are thus alternated in opposition
- A fourth strip W2 comprising eight B patterns each consisting of one Satin weave 6 in the longitudinal direction;
- A fifth strip W1 comprising, in the longitudinal direction, three A patterns each consisting of one Satin 8 weave and three C patterns each consisting of four Satin weaves 4 arranged in a square, the patterns A and C being alternated; as in the first strip.

This sequence of strips W1 - W2 - W3 - W2 - W1 provides a flexible fabric having a very uniform distribution of light. More details of such a structure are given in WO 2012/098488.

The weft density of polymethyl methacrylate optical fibres is set to 37 cm⁻¹.

Figure 3 explains differences among SW4, SW6 and SW8 by showing the structure of the fabric with vertically warp polyester yarns and horizontally weft polymethyl methacrylate optical fibres yarns. Black areas indicate when a polymethyl methacrylate optical fibre is above the polyester yarns and white areas when polyester yarns are above polymethyl methacrylate optical fibres in the fabric.

### Example 2: Manufacture of a medical device for photodynamic therapy comprising individually manageable areas of light emission

### Step A:

The 15 cm wide light diffuser textile of example 1 comprises 555 optical fibres. A medical device having the structure shown on figure 2, comprising three light diffuser textiles was manufactured as follows: Starting from one side of the textile, three bundles of 185 adjacent optical fibres extending from each side of each of the diffuser textiles (each group corresponding to 5 cm wide woven fibres) were prepared. Total of six bundles for both sides.

### Step B:

Each pair of bundles of each light diffuser textile was inserted into a ferrule of highly polished brass whose internal diameter is adapted to the number of individual fibres inserted therein (internal diameter 5 mm for 370 fibres - 185 from each side - of 250 µm diameter). Three ferrules were used for the six bundles. This arrangement allows provision of light from both sides of a light diffuser textile with a same source of light.

Araldite 2011 epoxy adhesive from Huntsman Advanced Materials is used to bond the optic fibres into the ferrule. 12 hours later, after hardening of the glue, the excess length of the optical fibres was cut and polished at the extremity of the ferrule. Polishing requires 99% isopropyl alcohol, polishing (lapping) film and pad, a polishing puck. Special polishing paper is employed successively from coarser grits to finer grits measured in microns. The last is a 0.5µm polishing paper.

After fine polishing of the extremity of the ferrule, a medical device provided with three connectors was thus obtained.

### Example 3: Manufacture of a medical device for photodynamic therapy comprising individually manageable areas of light emission

A medical device having the structure shown on figure 2, comprising three light diffuser textiles was manufactured as explained in example 2 with a difference that one ferrule was used for each of the six bundles. Six ferrules were thus used for the six bundles. A ferrule of internal diameter 3.56mm mm was used for the 185 fibres of each bundle.

This arrangement allows provision of light from both sides of a light diffuser textile using two sources of light.

### Example 4: Manufacture of a medical device for photodynamic therapy comprising individually manageable areas of light emission

A medical device having the structure shown on figure 1, comprising three light diffuser textiles was manufactured as explained in example 2 with a difference that a single bundle was prepared for each of the three light diffuser textiles

One ferrule of internal diameter 3.56mm mm was used for the 185 fibres of each bundle. Three ferrules were thus used for the three bundles. This arrangement allows provision of light from only one side of a light diffuser textile.

In the above examples 2, 3 and 4, after hardening of the glue, the excess length of the optical fibres was cut and polished using a fine grit (5 µm) abrasive paper. Special polishing paper is employed successively from coarser grits to finer grits measured in microns. The last is a 0.5µm polishing paper.

### Example 5: Manufacture of a medical apparatus for photodynamic therapy comprising a medical device of the invention and a light-emitting device

The ferrules of each of the medical devices of examples 2, 3 and 4 were connected to a 635 nm laser diode (5W, Dilas, Germany).

The medical device of example 2 is described in details. The light diffuser textile used is the specific textile of example 1.

Each light diffuser textile is equipped with a single connector (or ferrule) of 5.02 mm internal diameter which includes 370 fibres of diameter 250µm. One laser diode commercialised by DILAS (reference M1F4S22-638.3-5C-SS2.6) is used to illuminate each light diffuser textile. This laser diode emits 5 W. it is equipped with a standard SMA connector. (ref DS11-lp-o8617-v0). The diode is mounted on a Peltier module (PE1-12707AC society Multicomp) to ensure the cooling of the diode.

The diode and the Peltier module are supplied via a power supply OSTECH (ref DS11-lp-o8617). This power supply manages all functions of the diode and Peltier module, using a computer with a RS-232 connection. The control software is provided by OSTECH. It is therefore possible to manage the diode and Peltier module through a list of commands provided by OSTECH with a laptop at the convenience of the user. See http://www.ostech.com/home_en.htm and http://www.ostech.com/downloads_software_en.htm

A 3 m long optical fibre of 600µm equipped with a SMA connector at each end (supplied by Sedi http://www.sedi-fibres.com/medical_225.html fibre) is connected to an optical device for expanding the 600µm beam to 5mm.

The three light diffuser textiles are provided with identical laser diodes. A software written in Labview allows managing and controlling the three laser diodes via the RS-232.

The software allows to independently set each diode power (0 to 5 W), the time of illumination (1 sec to 99 minutes), the delay of illumination (1s to 99 minutes) and the number illuminations (1-99). After programming each laser diode, it is possible to synchronize the operation of the diodes.

### Example 6: Manufacture of a medical device for photodynamic therapy comprising individually manageable areas of light emission and means for assessment of bleaching of a sensitizer

As indicated above in example 3, three individually manageable areas of light emission were produced with a difference that for each area the optical fibres were bundled as follows: a first bundle comprising every fifth optical fibre was prepared. A second bundle was prepared comprising the remaining optical fibres. Accordingly a first bundle comprises 1/5 of the optical fibres whereas the second bundle comprises 4/5 of the optical fibres. The first bundle may be used for assessment of the bleaching of a photosensitiser whereas the second bundle may be used for phototherapeutic treatments.

This medical device comprises six bundles, each provided with a ferrule: three for assessment of the bleaching of a photosensitiser and three for phototherapeutic (or photodynamic) treatments.

### EXPERIMENTAL DATA

The experiments were implemented with light diffuser textiles having the specific structure described in Example 1.

### Experimentation 1: Sequence of illumination

In reference to example 5: The first laser diode is switched on. When the first laser diode has completed a 1 minute illumination, it is switched off. An instruction of switching on is sent to the second laser diode. After completion of a 1 minute illumination by the second laser diode, said laser diode is switched off and a instruction of switching on is sent to the third laser diode, which after completion of a 1 minute illumination is switched off. The first laser diode is switched on for a second time and so on depending on the number of illuminations programmed by the user.

Therefore in the present case, laser diodes are scheduled to have a rest period of 2 minutes.

With a power of 10mW/cm², a treatment is implemented for the duration of one hour and a half such that 18 J/cm² of treated surface. Accordingly, one given light diffuser textile is illuminated for 30 min.

### Experimentation 2: Homogeneity of light delivery by the medical apparatus of example 2 (size 5 cm x 21.5 cm)

The measurement of the irradiance was achieved with a wattmeter OPHIR PD 300. The measuring head has a surface of 1 cm². It is therefore possible to obtain a measure of the irradiance expressed in mW / cm². The measurement was carried out on 105 areas of 1 cm² of one light diffuser textile. The distribution of the light emitted by the illuminated tissue was thus obtained. For an injected power of 5 W, the tested textile of the invention provides an intensity of 18.2 mW.cm² ± 2.5 mW.cm⁻². Therefore, a medical device of the invention provides a strong light.

The light delivered by the medical apparatus of example 5 was found to be remarkably homogeneous.

Figure 7 illustrates this homogeneous light diffusion.

### Experimentation 3. Measurement of the temperature reached by a medical light diffuser textile of the invention in the course of a treatment (size: 5 cm x 21.5 cm)

Temperature evolution of a medical light diffuser textile as a function of duration of illumination was controlled using an infrared thermographic camera (Fluke Ti 125). After 10 min of illumination with an irradiance of 18.2 mW/cm², the increase of temperature was only 0.6 °C.

The homogeneity of illumination achieved on an irregular surface with hollows and bumps is much better than results obtained with commercially available inflexible LED panels.

### Experimentation 4: Measurement of bleaching of a photosensitiser

As indicated above in example 3, three individually manageable areas of light emission were produced with a difference that for each area the optical fibres were bundled as follows: a first bundle comprising every fifth optical fibre was prepared. This first bundle has a diameter of 2.25 mm and comprises 74 fibres. A second bundle was prepared comprising the remaining optical fibres. Accordingly a first bundle comprises 1/5 of the optical fibres whereas the second bundle comprises 4/5 of the optical fibres. The first bundle may be used for assessment of the bleaching of a photosensitiser whereas the second bundle may be used for phototherapeutic treatments.

The first bundle is connected to a spectrometer 350-1100 nm USB2000+VIS-NIR (Ocean Optics Inc., Dunedin, FL, USA) having a high sensitivity at 705 nm using an optical system consisting of Thorlabs Inc. parts.

The spectrophotometer is connected to a laptop by an USB cable.

The illumination bundle comprising 296 fibres has a diameter of 4.51 mm and is connected to a laser diode as previously described.

The photosensitiser used is protoporphyrin IX. The photosensitiser was excited at 630 nm. Fluorescence emitted by the photosensitiser was measured at 705 nm.

A computer with a Labview software allows to manage a just-in-time the operation of illumination and acquisition of fluorescence spectra. The power of the laser diode emitting at 630nm is adjusted for obtaining an irradiance of 1mW/cm² in the red which corresponds to a power of 0.3 W. The Ocean Optics spectrophotometer is programmed for acquiring hundred fluorescence spectra (Integration time set to 0.5 seconds), i.e. 50 seconds. See http://www.oceanoptics.com/products/usb2000+precon.asp.

At the end of the acquisition of the data, the software calculates the intensity of fluorescence at 705nm. The initial value is the reference 100%. During photobleaching of the photosensitiser, the initial value decreases

If the fluorescence measurement is greater than 10% of the initial fluorescence, the treatment is continued. The spectrophotometer is stopped. The management software of the red laser diode switches on to treatment and increases the power to 3 W to achieve an irradiance of 10mW/cm², considered therapeutic.

After the scheduled duration of treatment (one minute), the fluorescence measurement is performed again as described above.

When the fluorescence is 10% of the initial value, the treatment procedure is interrupted.

### Experimentation 5: Amount of light collected by a medical light diffuser textile of the invention (size 5 cm x 21.5 cm)

Two identical medical devices described in examples 2 and 3 (21.5 X 5 cm) were used for the experiment.

The first one was used as light emitter. Its optical connector was connected to a Modulight laser 1,3W emitting at 635nm equipped with a THORLABS coupling Tube.

The second one was superimposed onto the first one and was used as light collector for the measurement of the input light. The amount of input light is the same on both sides of the light emitter. Its optical connector was connected to a Newport wattmeter 841-PE with a PD 300 measure head (PhotoDetector)via a THORLABS coupling Tube.

An OPHIR wattmeter with PD 300 measure head was used for the measurement of the input light.

Sedi SMA-SMA cords were used for the connexions.

A power of 240 mw was measured by the OPHIR wattmeter. A power of 192 µw was measured by the more sensitive Newport wattmeter ((PhotoDetector)

### Conclusion:

The medical light diffuser textile is able to collect and also to detect light.

## Claims

1. A medical apparatus for photodynamic therapy comprising:
- a medical device comprising a flexible light source wherein said flexible light source comprises 3 individually manageable areas (2, 3, 4) of light emission and wherein each area (2, 3, 4) comprises a light diffuser textile comprising optical fibres (10) adapted to provide side diffusion of a light, wherein the light diffuser textile is obtained by weaving and comprises a weft (10) comprising optical fibres and a warp (13),
- a light-emitting device (7) suitable for photodynamic therapy, the light-emitting device (7) being connected to the optical fibres providing side diffusion of a light,
- a computer for managing light emission, **characterised by**
the computer being configured to sequentially put on each of the 3 individually manageable areas for a 1 minute illumination and switch off said individually manageable area.

2. The medical apparatus according to claim 1, wherein the weft comprises polymethyl methacrylate optical fibres.

3. The medical apparatus according to any of claims 1 and 2, wherein the warp comprises polyester yarns.

4. The medical apparatus according to one of claims 1 to 3, wherein optical fibres (10) constituting the weft of each light diffuser textile are collected as bundles and wherein said bundles are further provided with a port of entry of light.

5. The medical apparatus according to any of claims 1 to 4, wherein the weft (10) of each light diffuser textile is further provided with one or several ports (6) of entry of light.

6. The medical apparatus according to any of claims 1 to 5 and further comprising a photodetector, wherein the medical device is bifunctional, wherein the 3 individually manageable areas of light emission comprising optical fibres providing side absorption of a light connected to the photodetector.

7. The medical apparatus of claim 6, wherein each light diffuser textile is provided with two bundles of optical fibres wherein optical fibres of each bundle are different of optical fibres of the other bundle.

8. The medical apparatus according to claim 7, wherein each bundle is provided a port (6) of entry of light.

9. The medical apparatus according to any of claims 7 or 8, wherein the ratio fibres used for detection / fibres used for therapy is in the range 1/2 to 1/15.

10. A process for the manufacture of a medical apparatus according to any of claims 1 to 9, comprising the steps consisting in manufacturing 3 light diffuser textiles obtained by:
- providing a warp (13)
- interlacing at right angles a weft comprising optical fibres (10) and said interlacing providing the bending of said weft (10) such that a critical angle allowing side emission of light is exceeded, and
- preparing a flexible light source comprising said 3 light diffuser textiles,
- providing a light-emitting device (7) suitable for photodynamic therapy and connecting the light-emitting device (7) to the optical fibres providing side diffusion of a light,
- providing a computer for managing light emission, the computer being configured to sequentially put on each of the 3 individually manageable areas for a 1 minute illumination and switch off said individually manageable area.

## Patentansprüche

1. Ein medizinischer Apparat für eine photodynamische Therapie, umfassend:
- eine medizinische Vorrichtung, umfassend eine flexible Lichtquelle, wobei die flexible Lichtquelle 3 individuell kontrollierbare lichtemittierende Bereiche (2, 3, 4) umfasst, und wobei jeder Bereich (2, 3, 4) ein lichtstreuendes Textil umfasst, das optische Fasern (10) umfasst, die eingerichtet sind, um seitliche Lichtstreuung bereitzustellen, wobei das lichtstreuende Textil durch Weben erhalten wird und einen Kettfaden (10), der optische Fasern umfasst, und einen Schussfaden (13) umfasst,
- eine Licht emittierende Vorrichtung (7), die für photodynamische Therapie geeignet ist, wobei die Licht emittierende Vorrichtung (7) mit den optischen Fasern verbunden ist, die die seitliche Lichtstreuung bereitstellen,
- einen Computer zum Kontrollieren der Lichtemission, **dadurch gekennzeichnet, dass** der Computer dazu konfiguriert ist, jeden der 3 individuell kontrollierbaren Bereiche sequentiell für eine 1-minütige Beleuchtung einzuschalten und den individuell kontrollierbaren Bereich abzuschalten.

2. Der medizinische Apparat nach Anspruch 1, wobei der Kettfaden optische Polymethylmethacrylatfasern umfasst.

3. Der medizinische Apparat nach einem der Ansprüche 1 und 2, wobei der Schussfaden Polyestergarne umfasst.

4. Der Medizinische Apparat nach einem der Ansprüche 1 bis 3, wobei optische Fasern (10), die den Kettfaden jedes lichtstreuenden Textils bilden, als Bündel zusammengenommen sind, und wobei die Bündel ferner mit einem Lichteintrittanschluss ausgestattet sind.

5. Der Medizinische Apparat nach einem der Ansprüche 1 bis 4, wobei der Kettfaden (10) jedes lichtstreuenden Textils ferner mit einem oder mehreren Lichteintrittanschlüssen (6)ausgestattet ist.

6. Der medizinische Apparat nach einem der Ansprüche 1 bis 5 und ferner umfassend einen Photodetektor, wobei die medizinische Vorrichtung bifunktional ist, wobei die 3 individuell kontrollierbaren lichtemittierenden Bereiche, die optische Fasern umfassen, die seitliche Lichtabsorption bereitstellen, mit dem Photodetektor verbunden sind.

7. Der medizinische Apparat nach Anspruch 6, wobei jedes lichtstreuende Textil mit zwei Bündeln von optischen Fasern ausgestattet ist, wobei optische Fasern von jedem Bündel sich von optischen Fasern des anderen Bündels unterscheiden.

8. Der medizinische Apparat nach Anspruch 7, wobei jedes Bündel mit einem Lichteintrittanschluss (6) ausgestattet ist.

9. Der medizinische Apparat nach einem der Ansprüche 7 oder 8, wobei das Verhältnis zur Detektierung verwendete Fasern / zur Therapie verwendete Fasern im Bereich von 1/2 bis 1/15 liegt.

10. Ein Verfahren zur Herstellung eines medizinischen Apparats nach einem der Ansprüche 1 bis 9, umfassend die Schritte, die aus der Herstellung von 3 lichtstreuenden Textilien bestehen, die erhalten werden durch:
- Bereitstellen eines Kettfadens (13);
- Durchwirken eines Schussfadens, der optische Fasern (10) umfasst, in rechten Winkeln, und wobei das Durchwirken die Biegung des Schussfadens (10) derart bereitstellt, dass ein kritischer Winkel, der seitliche Lichtemission gestattet, überschritten wird, und
- Herstellen einer flexiblen Lichtquelle, die die 3 lichtstreuenden Textilien umfasst,
- Bereitstellen einer Licht emittierenden Vorrichtung (7), die für photodynamische Therapie geeignet ist, und Verbinden der Licht emittierenden Vorrichtung (7) mit den optischen Fasern, die seitliche Lichtstreuung bereitstellen,
- Bereitstellen eines Computers zum Kontrollieren der Lichtemission, wobei der Computer dazu konfiguriert ist, jeden der 3 individuell kontrollierbaren Bereiche sequentiell für eine 1-minütige Beleuchtung einzuschalten und den individuell kontrollierbaren Bereich abzuschalten.

## Revendications

1. Appareil médical pour thérapie photodynamique comprenant :
- un dispositif médical comprenant une source de lumière flexible dans lequel ladite source de lumière flexible comprend 3 zones pouvant être gérées individuellement (2, 3, 4) d'émission de lumière et dans lequel chaque zone (2, 3, 4) comprend un textile diffuseur de lumière comprenant des fibres optiques (10) adaptées pour fournir une diffusion latérale d'une lumière, dans lequel le textile diffuseur de lumière est obtenu par tissage et comprend une trame (10) comprenant des fibres optiques et une chaîne (13),
- un dispositif électroluminescent (7) approprié pour une thérapie photodynamique, le dispositif électroluminescent (7) étant connecté aux fibres optiques fournissant une diffusion latérale d'une lumière,
- un ordinateur pour gérer une émission de lumière,
**caractérisé par** l'ordinateur étant configuré pour allumer séquentiellement chacune des 3 zones pouvant être gérées individuellement pendant 1 minute d'éclairage et éteindre ladite zone pouvant être gérée individuellement.

2. Appareil médical selon la revendication 1, dans lequel la trame comprend des fibres optiques de polyméthacrylate de méthyle.

3. Appareil médical selon l'une quelconque des revendications 1 et 2, dans lequel la chaîne comprend des fils de polyester.

4. Appareil médical selon l'une quelconque des revendications 1 à 3, dans lequel des fibres optiques (10) constituant la trame de chaque textile diffuseur de lumière sont rassemblées en faisceaux et dans lequel lesdits faisceaux sont en outre dotés d'un orifice d'entrée de lumière.

5. Appareil médical selon l'une quelconque des revendications 1 à 4, dans lequel la trame (10) de chaque textile diffuseur de lumière est en outre dotée d'un ou de plusieurs orifices (6) d'entrée de lumière.

6. Appareil médical selon l'une quelconque des revendications 1 à 5 et comprenant en outre un photodétecteur, dans lequel le dispositif médical est bifonctionnel, dans lequel les 3 zones pouvant être gérées individuellement d'émission de lumière comprenant des fibres optiques fournissant une absorption latérale d'une lumière connectée au photodétecteur.

7. Appareil médical selon la revendication 6, dans lequel chaque textile diffuseur de lumière est doté de deux faisceaux de fibres optiques dans lequel des fibres optiques de chaque faisceau sont différentes des fibres optiques de l'autre faisceau.

8. Appareil médical selon la revendication 7, dans lequel chaque faisceau est doté d'un orifice (6) d'entrée de lumière.

9. Appareil médical selon l'une quelconque des revendications 7 ou 8, dans lequel le rapport entre des fibres utilisées pour la détection/des fibres utilisées pour la thérapie se situe dans la plage de 1/2 à 1/15.

10. Procédé pour la fabrication d'un appareil médical selon l'une quelconque des revendications 1 à 9, comprenant les étapes consistant à la fabrication de 3 textiles diffuseurs de lumière obtenus par :
- la fourniture d'une chaîne (13)
- l'entrelacement à angle droit d'une trame comprenant des fibres optiques (10) et ledit entrelacement fournissant la courbure de ladite trame (10) de sorte qu'un angle critique permettant une émission latérale de lumière soit dépassé, et
- la préparation d'une source de lumière flexible comprenant lesdits 3 textiles diffuseurs de lumière,
- la fourniture d'un dispositif électroluminescent (7) approprié pour une thérapie photodynamique et la connexion du dispositif électroluminescent (7) aux fibres optiques fournissant une diffusion latérale d'une lumière,
- la fourniture d'un ordinateur pour gérer une émission de lumière, l'ordinateur étant configuré pour allumer séquentiellement chacune des 3 zones pouvant être gérées individuellement pendant 1 minute d'éclairage et éteindre ladite zone pouvant être gérée individuellement.
